# EUROPÄISCHE PATENTSCHRIFT

(11) **EP 2 389 347 B1**
(45) Veröffentlichungstag und Bekanntmachung des Hinweises auf die Patenterteilung: **16.12.2015**
(21) Anmeldenummer: 10701346.8
(22) Anmeldetag: 19.01.2010
(51) Int. Cl.: C07C 29/80, C07C 29/60, C07C 31/20

(54) **VERFAHREN ZUR HERSTELLUNG VON DESODORIERTEM 1,2-PROPANDIOL**
METHOD FOR PRODUCING DEODORIZED 1,2-PROPANEDIOL
PROCÉDÉ DE PRODUCTION DE 1,2-PROPANEDIOL DÉSODORISÉ

(30) Priorität: 21.01.2009 EP 09151011
(43) Veröffentlichungstag der Anmeldung: 30.11.2011
(73) Patentinhaber: BASF SE, 67056 Ludwigshafen (DE)
(72) Erfinder: MAURER, Stephan, 67435 Neustadt-Gimmeldingen (DE); PROCHAZKA, Roman, 68163 Mannheim (DE); BEY, Oliver, 67150 Niederkirchen (DE); STEINER, Jochen, 64625 Bensheim (DE); HENKELMANN, Jochem, 68165 Mannheim (DE); THEIS, Gerhard, 67133 Maxdorf (DE); WAHL, Peter, 69118 Heidelberg (DE); HEIMANN, Frank, 67065 Ludwigshafen (DE)
(86) Internationale Anmeldenummer: PCT/EP2010/050597
(87) Internationale Veröffentlichungsnummer: WO 2010/084118

(56) Entgegenhaltungen:
- WO-A1-2004/041759
- WO-A1-2008/020077
- DASARI M A ET AL: "Low-pressure hydrogenolysis of glycerol to propylene glycol" APPLIED CATALYSIS A: GENERAL, ELSEVIER SCIENCE, AMSTERDAM, NL, Bd. 281, Nr. 1-2, 18. März 2005 (2005-03-18), Seiten 225-231, XP025332894 ISSN: 0926-860X [gefunden am 2005-03-18]

## Beschreibung

Die vorliegende Erfindung betrifft ein Verfahren zur Herstellung von desodoriertem 1,2-Propandiol durch destillative Aufarbeitung des bei der Hydrierung von Glycerin erhaltenen Hydrieraustrags.

Verfahren zur Herstellung von Rohstoffen und insbesondere Kraftstoffen aus biogenen fett- oder ölhaltigen Ausgangsgemischen sowie beispielsweise in Restaurants anfallenden gebrauchten Ölen und tierischen Fetten sind seit längerem bekannt, wobei vorzugsweise Rapsöl als Ausgangsprodukt bei der Produktion biogener Kraftstoffe eingesetzt wird. Biogene Öle und Fette selbst sind als Motorenkraftstoff weniger geeignet, da sie zuvor durch meist aufwendige Verfahren gereinigt werden müssen. Dazu zählt die Entfernung von Lecithinen, Kohlenhydraten und Proteinen, die Entfernung des so genannten Ölschlamms sowie die Entfernung der beispielsweise in Rapsöl in größeren Mengen enthaltenen freien Fettsäuren. So aufbereitete Pflanzenöle weichen dennoch von den technischen Eigenschaften herkömmlicher Dieselkraftstoffe in mehreren Punkten ab. So weisen sie in der Regel eine höhere Dichte als Dieselkraftstoff auf, die Cetan-Zahl von Rapsöl ist geringer als die von Dieselkraftstoff und die Viskosität ist gegenüber der von Dieselkraftstoffen um ein Vielfaches höher. Zur Lösung der damit verbundenen Probleme ist bekannt, die in den biogenen Öl- und Fettausgangsgemischen enthaltenen Triglyceride in Fettsäuremonoalkylester, insbesondere Methyl- oder Ethylester, umzuwandeln. Diese auch als "Biodiesel" bezeichneten Ester lassen sich in der Regel ohne größere Nachrüstungen in Dieselmotoren einsetzen. Bei der Umesterung der Triglyceride zur Biodieselherstellung fällt auch Glycerin an, aus welchem in an sich bekannter Weise durch Hydrierung 1,2-Propandiol gewonnen werden kann.

So beschreibt die DE-PS 524 101 ein solches Verfahren, bei dem man unter anderem Glycerin einer Gasphasenhydrierung in Gegenwart eines Hydrierungskatalysators mit Wasserstoff in erheblichem Überschuss unterzieht, wobei mit Brom aktivierte Kupfer- oder Kobaltkatalysatoren eingesetzt werden. Die DE 4302464 A1 beschreibt ein Verfahren zur Herstellung von 1,2-Propandiol durch Hydrierung von Glycerin in Gegenwart eines heterogenen Katalysators, wobei man Glycerin über ein Katalysatorbett aus kupferhaltigem Katalysator leitet. Die PCT/EP2007/0511983 beschreibt ein Verfahren zur Herstellung von 1,2-Propandiol, bei dem man einen glycerinhaltigen Strom einer Hydrierung in Gegenwart eines kupferhaltigen, heterogenen Katalysators unterzieht.

Die hierbei verwendeten glycerinhaltigen Ströme können noch zahlreiche unerwünschte Komponenten enthalten wie Schwefelsäure, Schwefelwasserstoff, Thioalkohole, Thioether, Kohlenoxidsulfid und beispielsweise stickstoffhaltige Verbindungen, z. B. Aminosäuren. Obwohl bekannt ist, diese Verunreinigungen bereits vor der Hydrierung des Glycerins zu entfernen, sind diese zum Teil noch nach der Hydrierung des Glycerins im 1,2-Propandiol vorhanden. Derartige Verunreinigungen können dazu führen, dass das erhaltene 1,2-Propandiol wegen des von ihnen ausgehenden Geruchs für bestimmte Anwendungen nicht in Frage kommt. Insbesondere stören Verunreinigungen durch Thiole, Fettsäuren, Ester, Aldehyde und Ketone, die als geruchsintensive Substanzen teilweise schon im ppb-Bereich wahrgenommen werden. Die WO 2008/020077 offenbart ein Verfahren zur Herstellung von 1,2-Propandiol mit einer Reinheit von mindestens 98%, wobei in einem ersten Schritt Glycerin in Gegenwart eines heterogenen kupferhaltigen Katalysators kontinuierlich hydriert wird, wobei Glycerin zu höchstens 95 % umgesetzt wird, und die im ersten Schritt erhaltene Reaktionsmischung in einem zweiten Schritt einer Destillation unterworfen wird.

Der Erfindung liegt die Aufgabe zugrunde, ein Verfahren zur Herstellung von desodoriertem 1,2-Propandiol zur Verfügung zu stellen, welches insbesondere zur Aufarbeitung von Propandiol geeignet ist, welches durch Hydrierung von Glycerin, welches seinerseits aus natürlichen Rohstoffen, insbesondere Rapsöl, stammt, erhalten wird.

Gefunden wurde nun ein Verfahren zur Reinigung von 1,2-Propandiol von Verunreinigungen durch Destillation, dadurch gekennzeichnet, dass das verunreinigte Propandiol
1. zunächst in eine Leichtsiederkolonne A gefahren wird, die einen Verstärkungsteil und einen Abtriebsteil umfasst, wobei die Leichtsiederkolonne A im Verstärkungsteil weniger als 20 theoretische Trennstufen und im Abtriebsteil weniger als 40 theoretische Trennstufen aufweist, das Rücklaufverhältnis kleiner als 5 ist und die Sumpftemperatur unter 200 °C liegt
   1.1 über Kopf der Kolonne die Leichtsieder, insbesondere Methanol, 1-Propanol, 2-Propanol, Wasser sowie leichtsiedende Geruchsstoffe abgetrennt werden und
   1.2 über Sumpf der Kolonne ein noch Verunreinigungen enthaltender 1,2-Propandiolstrom abgezogen wird, der aus wenigstens 97 Gew.-% 1,2-Propandiol besteht, der
2. in eine Kolonne B zur Reindestillation eingetragen wird, wobei
   2.1 gereinigtes, geruchsarmes 1,2-Propandiol die Kolonne als flüssiger Seitenabzug verlässt
   2.2 über Kopf der Kolonne B ein vorzugsweise geringer Strom an noch verunreinigten geruchshaltigen 1,2-Propandiol abgezogen wird und
   2.3 Schwersieder wie Ethylenglykol und Glycerin über Sumpf abgetrennt werden, wobei das zu reinigende 1,2-Propandiol aus dem Hydrieraustrag, der bei der Hydrierung von Glycerin zu 1,2-Propandiol anfällt, stammt.

Das noch verunreinigte 1,2-Propandiol wird in eine Leichtsiederkolonne A eingefahren, die mit dem Fachmann bekannten Einbauten versehen ist. Diese enthält im Verstärkungsteil weniger als 20 theoretische Trennstufen, typischerweise weniger als 5 theoretische Trennstufen. Der Abtriebsteil umfasst weniger als 40 theoretische Trennstufen, typischerweise weniger als 15 theoretische Trennstufen. Das Rücklaufverhältnis ist kleiner als 5, vorzugsweise kleiner als 1. Die Sumpftemperatur der Leichtsiederkolonne A liegt bei kleiner als 200 °C, vorzugsweise bei kleiner als 180 °C. Über Kopf der Leichtsiederkolonne A können vorzugsweise Leichtsieder wie Methanol, 1-Propanol, 2-Propanol und Wasser sowie leichtsiedende Geruchsstoffe abgetrennt werden. Der über Sumpf der Kolonne abgezogene noch Geruchstoff-haltige 1,2-Propandiolstrom kann noch schwersiedende Komponenten wie Ethylenglykol und Glycerin enthalten.

In der Kolonne zur Reindestillation B werden insbesondere Ethylenglykol und Glycerin im Wesentlichen über den Sumpf abgetrennt. Der gereinigte geruchsarme 1,2-Propandiolproduktstrom verlässt die Kolonne als flüssiger Seitenabzug mit vorzugsweise niedriger Ethylenglykolkonzentration von insbesondere kleiner 2000 Gew.-ppm, besonders bevorzugt kleiner 500 Gew.-ppm. Über Kopf der Kolonne kann ein vorzugsweise geringer Strom an noch geruchshaltigen 1,2-Propandiol mit gegebenenfalls geringer Ethylenglykolkonzentration abgezogen werden, der zur Geruchsausschleusung vollständig oder teilweise wieder zur Leichtsiederkolonne A zurückgefahren werden kann.

In einer bevorzugten Ausführungsform handelt es sich bei dem aus Kolonne A abgezogenen noch verunreinigten Propandiol um ein Gemisch, welches aus wenigstens 97 Gew.-% 1,2-Propandiol besteht, bezogen auf das Gesamtgewicht. Das aus der Kolonne B abgezogene gereinigte Propandiol weist vorzugsweise eine Konzentration von wenigstens 99, vorzugsweise 99,5, insbesondere 99,7 Gew.-% Propandiol auf mit einem maximalen Anteil von geruchsintensiven Stoffen von 0,01, insbesondere 0,001 Gew.-% und einem maximalen Anteil von Ethylenglykol von 0,2, vorzugsweise 0,05 Gew.-%. Unter geruchsintensiven Stoffen werden hierbei insbesondere Thiole, Fettsäuren, Ester, Aldehyde und Ketone verstanden.

In dieser bevorzugten Ausführungsform sind im Verstärkungsteil der Kolonne B oberhalb des flüssigen Seitenabzugs weniger als 60 theoretische Trennstufen, besonders bevorzugt weniger als 40 theoretische Trennstufen vorhanden. Vorzugsweise sind zwischen Feedstelle und flüssigem Seitenabzug weniger als 120 theoretische Trennstufen, besonders bevorzugt weniger als 80 theoretische Trennstufen vorhanden. Der Abtriebsteil umfasst vorzugsweise weniger als 60 theoretische Trennstufen, insbesondere weniger als 40 theoretische Trennstufen. Das auf den Feed der Reindestillationskolonne B bezogene Rücklaufverhältnis am Kopf der Kolonne B beträgt vorzugsweise weniger als 10, besonders bevorzugt weniger als 6. Die Sumpftemperatur der Kolonne B liegt vorzugsweise bei weniger als 200 °C, besonders bevorzugt bei weniger als 180 °C.

Das zu reinigende 1,2-Propandiol stammt aus dem Hydrieraustrag der bei der Hydrierung von Glycerin zu 1,2-Propandiol unter Verwendung von vorzugsweise kupferhaltigen Katalysatoren anfällt, wobei das Glycerin insbesondere aus nachwachsenden Rohstoffen wie Rapsöl gewonnen wird.

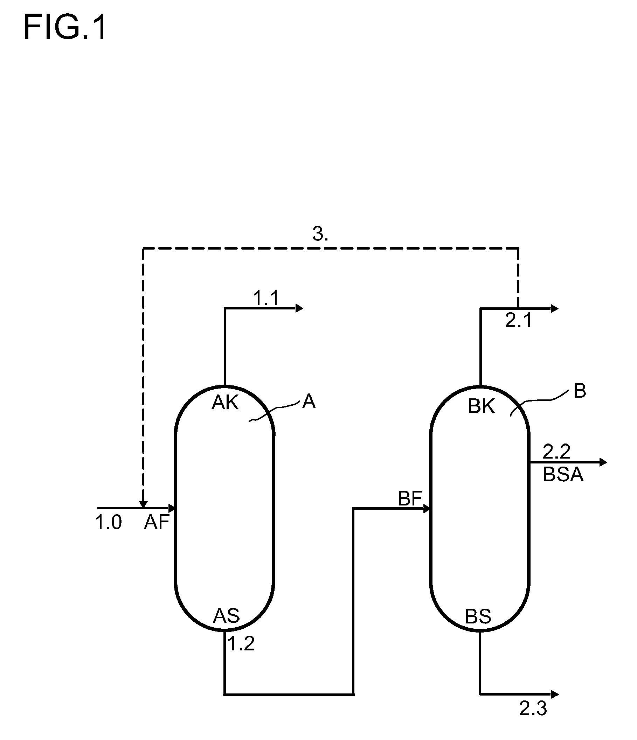

Figur 1 zeigt den schematischen Aufbau einer Kolonnenverschaltung, die für das erfindungsgemäße Verfahren vorteilhaft ist. Über eine Zuleitung 1.0 erfolgt die Zuführung von verunreinigtem Propandiol in die Leichtsiederkolonne A, die einen Kolonnenkopf AK und einen Kolonnensumpf AS aufweist. Über Leitung 1.1 können die Leichtsieder von Kolonnenkopf AK abgezogen werden, über den Abfluss 1.2 wird das noch verunreinigte Propandiol aus dem Kolonnensumpf AS abgezogen und über den Feed BF in die Kolonne B eingeleitet. Die Kolonne B weist einen Kolonnenkopf BK auf, aus welchem über Leitung 2.1 noch verunreinigtes Propandiol abgezogen wird, welches über die Leitung 3 zurückgeleitet werden kann in den verunreinigten Propandiolstrom 1.0.

Über Leitung 2.3 können die Schwersieder aus dem Kolonnensumpf BS abgezogen werden, das gereinigte Propandiol verlässt die Kolonne B über den Seitenabzug BSA (Leitung 2.2.).

Die Destillationskolonnen A und B können ganz oder teilweise Packungen aus geordneten oder ungeordneten Trennelementen enthalten, an denen der zwischen Sumpf und Kopf aufsteigende Dampf und die abströmende Flüssigkeit in innige Berührung gebracht werden. Als Trenneinbauten können neben regellosen Schüttungen zum Beispiel aus Raschig-Ringen, Pall-Ringen, Keramiksätteln auch geordnete Packungen eingesetzt werden. Diese können beispielsweise aus perforierten Metallblechen, aus Metallgeweben oder auch aus Kunststoff oder keramischem Material gefertigt sein. Die Destillationskolonnen können auch Böden aufweisen, beispielsweise Siebböden, Glockenböden, Ventilböden und Tunnelböden. Besonders bevorzugte Böden sind Thormannböden.

In einer besonders bevorzugten Ausführungsform weist die Destillationskolonne A im Verstärkerteil Böden, insbesondere Thormannböden und im Abtriebsteil eine geordnete Packung auf. In einer weiteren bevorzugten Ausführungsform ist die Kolonne B vollständig eine Packungskolonne mit einer Füllkörperpackung oder einer strukturierten Packung.

Die äußere Dimensionierung der Destillationskolonnen ist in Abhängigkeit der vorgenommenen Einbauten variabel; bei technischer Realisierung haben die Destillationskolonnen in einer bevorzugten Ausführungsform folgende Dimensionen:
Destillationskolonne A: Durchmesser 1 bis 3 m, Höhe 10 bis 30 m
Destillationskolonne B: Durchmesser 3,5 bis 5 m, Höhe 40 bis 80 m.

Es sind aber auch Dimensionierungen außerhalb dieser Bereiche möglich.

In einer Ausführungsform der Erfindung können insbesondere in Kolonne B, gegebenenfalls aber auch in Kolonne A Destillationshilfsstoffe, insbesondere Schleppmittel eingesetzt werden, um die destillative Abtrennung der Verunreinigungen von 1,2-Propandiol zu verbessern. Geeignete Hilfsstoffe sind beispielsweise Benzol, Toluol und Xylol. Diese Hilfsstoffe können beispielsweise in einer Menge von vorzugsweise unter 10 %, insbesondere unter 1 % und besonders bevorzugt im ppm-Bereich, bezogen auf das zu destillierende Gut in wenigstens einer der beiden Kolonnen, insbesondere in Kolonne B eingesetzt werden.

In einer weiteren bevorzugten Ausführungsform wird das erfindungsgemäße Verfahren in wenigstens einer der beiden Kolonnen A und B in an sich aus der WO 97/27164 A 1 bekannten Verfahren in Gegenwart einer reduzierenden Phosphorverbindung durchgeführt. Dieses kann durch eine Oberflächenbehandlung der Innenflächen der Anlagenteile erfolgen und diskontinuierlich oder kontinuierlich erfolgen. Bei diskontinuierlicher Arbeitsweise können die Anlagen vor der Destillation ganz oder teilweise mit der Phosphorverbindung behandelt werden. Bei kontinuierlicher Arbeitsweise kann dem Produktstrom die Phosphorverbindung zugesetzt werden. Erreicht wird hierdurch eine deutliche Reduzierung des Aldehydgehalts. Bevorzugte Phosphorverbindungen können anorganische oder organische Phosphorverbindungen oder eine Kombination daraus sein. In einer bevorzugten Ausführungsform handelt es sich um eine Phosphorverbindung mit dreiwertigem Phosphor.

Bevorzugt sind Phosphorige Säure (welche überwiegend in der stabilen tautomeren Form HP(O)(OH)₂ vorliegt) und Salze davon. Salze der Phosphorigen Säure sind vorzugsweise ausgewählt unter wasserlöslichen Salzen, wie insbesondere Alkalimetall-, Zink- und Calcium-Phosphiten. Besonders bevorzugte Alkalimetallphosphite sind Natrium- und Kaliumphosphit. Die entsprechenden Hydrogenphosphite sind ebenfalls verwendbar. Am meisten bevorzugt ist jedoch die Verwendung der Säure selbst.

Erfolgt die Zugabe der Phosphorverbindung zum Gemisch selbst, wird diese gewöhnlich so durchgeführt, dass die Phosphorverbindung in einem Anteil von etwa 10 bis etwa 5000 ppm vorliegt, bevor man Glykol aus dem Gemisch entfernt. Vorzugsweise sollte der Anteil im Bereich von etwa 100 bis etwa 1000 ppm, und insbesondere bei etwa 500 ppm liegen.

Das erfindungsgemäße gereinigte Propandiol eignet sich aufgrund seiner Geruchsarmut grundsätzlich für alle Zwecke, bei denen ein 1,2-Propandiol eingesetzt wird, insbesondere Feuchtmittel, Klebstoffe, Kühlflüssigkeiten und insbesondere Lebensmittel, Pharmazeutika und Kosmetika.

### Beispiel

Die Destillation eines verunreinigten Propandiols wurde in einer Kolonnenverschaltung gemäß Figur 1 durchgeführt. Als Leichtsiederkolonne A wurde eine Laborkolonne mit einem Innendurchmesser von 50 mm im Verstärkungsteil und 43 mm im Abtriebsteil verwendet. Bei den Einbauten handelte es sich um 10 Glockenböden zur Lösung der Trennaufgabe im wässrigen Verstärkungsteil und einer Montz-Packung A3 750 im Abtriebsteil. Die Packungshöhe im Abtriebsteil betrug 0,64 m.

Die Beheizung der Kolonne am Sumpf erfolgte mit einem Dünnschichtverdampfer, die Kopfkondensation durch Kühlwasser. Das Abgas (Temperatur ca. 25 °C am Kopfkondensator) wurde über mit Trockeneis gekühlte Kühlfallen gefahren. Die Kolonne war begleitbeheizt und wurde bei einer Sumpftemperatur von 153 °C und einem Kopfdruck von 300 mbar abs. betrieben.

Die Reindestillation wurde in einer Kolonne B durchgeführt, die einen Innendurchmesser von 64 mm aufwies. Die Feedzugabe erfolgte bei 1,64 m Packungshöhe von unten gerechnet. Bei 4,55 m Packungshöhe wurde das reine 1,2-Propandiolprodukt als flüssiger Seitenstrom entnommen. Die gesamte Packungshöhe betrug 5,85 m Montz A3 750.

Die Beheizung der Kolonne am Sumpf erfolgte mit einem metallischen Fallfilmverdampfer, die Kopfkondensation mit Kühlwasser. Das Abgas (Temperatur ca. 25 °C ex Kopfkondensator) wurde über Trockeneis-gekühlte Fallen gefahren. Die Kolonne war begleitgeheizt und wurde bei einer Sumpftemperatur von 153 °C und einem Kopfdruck von 200 mbar abs. betrieben. Folgende Ergebnisse wurden erhalten:

| | Einheit | Leichtsiederkolonne A | | | Kolonne zur Reindestillation B | | |
|---|---|---|---|---|---|---|---|
| Kopfdruck | mbar abs. | 300 | | | 200 | | |
| Sumpftemperatur | °C | 153 | | | 153 | | |
| Rücklaufverhältnis | kg/kg | 0,8 | | | --- | | |
| Rücklauf- zu Feedmenge | kg/kg | --- | | | 3,9 | | |
| F-Faktor | Pa^{0,5} | 0,4 - 0,9 | | | 0,5 | | |

| Konzentrationen: | | Feed AF | Kopf AK | Sumpf AS | Kopf BF | Sumpf BS | Seitenabzug BSA |
|---|---|---|---|---|---|---|---|
| Wasser | Gew.-% | 25,40 | 95,60 | 0,02 | 0,27 | 0,02 | 0,03 |
| Methanol | Gew.-% | 0,40 | 1,50 | < 0,005 | < 0,005 | < 0,005 | < 0,005 |
| 2-Propanol | Gew.-% | 0,20 | 0,50 | < 0,005 | < 0,005 | < 0,005 | < 0,005 |
| 1-Propanol | Gew.-% | 0,70 | 2,70 | < 0,005 | < 0,005 | < 0,005 | < 0,005 |
| 1,2-Propandiol | Gew.-% | 72,10 | 0,02 | 98,70 | 99,60 | 13,00 | 99,90 |
| Ethylenglykol | Gew.-% | 0,80 | < 0,01 | 1,00 | < 0,01 | 52,90 | 0,046 |
| Glycerin | Gew.-% | 0,50 | < 0,02 | 0,60 | < 0,02 | 31,50 | < 0,02 |

## Patentansprüche

1. Verfahren zur Reinigung von 1,2-Propandiol von Verunreinigungen durch Destillation, **dadurch gekennzeichnet, dass** das verunreinigte Propandiol
1. zunächst in eine Leichtsiederkolonne A gefahren wird, die einen Verstärkungsteil und einen Abtriebsteil umfasst, wobei die Leichtsiederkolonne A im Verstärkungsteil weniger als 20 theoretische Trennstufen und im Abtriebsteil weniger als 40 theoretische Trennstufen aufweist, das Rücklaufverhältnis kleiner als 5 ist und die Sumpftemperatur unter 200 °C liegt 1.1
über Kopf der Kolonne die Leichtsieder, insbesondere Methanol, 1-Propanol, 2-Propanol, Wasser sowie leichtsiedende Geruchsstoffe abgetrennt werden und
1.2 über Sumpf der Kolonne ein noch Verunreinigungen enthaltender 1,2-Propandiolstrom abgezogen wird, der aus wenigstens 97 Gew.-% 1,2-Propandiol besteht, der
2. in eine Kolonne B zur Reindestillation eingetragen wird, wobei 2.1
gereinigtes, geruchsarmes 1,2-Propandiol die Kolonne als flüssiger Seitenabzug verlässt
2.2 über Kopf der Kolonne B ein vorzugsweise geringer Strom an noch verunreinigten geruchshaltigen 1,2-Propandiol abgezogen wird und
2.3 Schwersieder wie Ethylenglykol und Glycerin über Sumpf abgetrennt werden, wobei
das zu reinigende 1,2-Propandiol aus dem Hydrieraustrag, der bei der Hydrierung von Glycerin zu 1,2-Propandiol anfällt, stammt.

2. Verfahren nach wenigstens einem der vorgehenden Ansprüche, **dadurch gekennzeichnet, dass** in der Reindestillationskolonne B im Verstärkungsteil oberhalb des flüssigen Seitenabzugs weniger als 60 theoretische Trennstufen, zwischen Feedstelle und flüssigem Seitenabzug weniger als 120 theoretische Trennstufen und im Abtriebsteil weniger als 60 theoretische Trennstufen vorliegen und dass das auf den Feed der Reindestillationskolonne bezogene Rücklaufverhältnis am Kopf der Kolonne weniger als 10 beträgt und die Sumpftemperatur unter 200 °C liegt.

3. Verfahren nach wenigstens einem der vorhergehenden Ansprüche, **dadurch gekennzeichnet, dass** das in die Kolonne A eingeführte verunreinigte Propandiol mit Thiolen, Fettsäuren, Ester, Aldehyden und/oder Ketonen verunreinigt ist.

4. Verfahren nach wenigstens einem der vorhergehenden Ansprüche, **dadurch gekennzeichnet, dass** das gereinigte Propandiol nach Abzug aus der Reindestillationskolonne B zu wenigstens 99 Gew.-% aus reinem 1,2-Propandiol und zu maximal 0,01 Ges-% aus Thiolen, Fettsäuren, Estern, Aldehyden und/oder Ketonen und zu maximal aus 0,1 Gew.-% Ethylenglykol besteht.

5. Verfahren nach wenigstens einem der vorhergehenden Ansprüche, **dadurch gekennzeichnet, dass** ein verunreinigtes Propandiol gereinigt wird, welches erhalten wurde durch Hydrierung eines glycerinhaltigen Stromes in Gegenwart eines Katalysators.

## Claims

1. A process for purifying 1,2-propanediol to remove impurities by distillation, wherein the contaminated propanediol
1. is first conducted into a low boiler column A which comprises a rectifying section and a stripping section, the low boiler column A having fewer than 20 theoretical plates in the rectifying section and fewer than 40 theoretical plates in the stripping section, the reflux ratio being less than 5 and the bottom temperature being less than 200°C,
1.1 the low boilers, especially methanol, 1-propanol, 2-propanol, water and low-boiling odorous substances, being removed via the top of the column and
1.2 a 1,2-propanediol stream which still comprises impurities and consists of at least 97% by weight of 1,2-propanediol being drawn off via the bottom of the column, and being
2. introduced into a column B for purifying distillation,
2.1 purified, low-odor 1,2-propanediol leaving the column as a liquid side draw,
2.2 a preferably minor stream of still-contaminated odor-containing 1,2-propanediol being drawn off via the top of column B and
2.3 high boilers such as ethylene glycol and glycerol being removed via the bottom,
the 1,2-propanediol to be purified originating from the hydrogenation output obtained in the hydrogenation of glycerol to 1,2-propanediol.

2. The process according to at least one of the preceding claims, wherein, in the purifying distillation column B, there are fewer than 60 theoretical plates in the rectifying section above the liquid side draw, fewer than 120 theoretical plates between the feed point and the liquid side draw, and fewer than 60 theoretical plates in the stripping section, and wherein the reflux ratio at the top of the column, based on the feed of the purifying distillation column, is less than 10 and the bottom temperature is less than 200°C.

3. The process according to at least one of the preceding claims, wherein the contaminated propanediol introduced into column A is contaminated with thiols, fatty acids, esters, aldehydes and/or ketones.

4. The process according to at least one of the preceding claims, wherein the purified propanediol, after removal from the purifying distillation column B, consists to an extent of at least 99% by weight of pure 1,2-propanediol and to an extent of not more than 0.01% by weight of thiols, fatty acids, esters, aldehydes and/or ketones, and to an extent of not more than 0.1% by weight of ethylene glycol.

5. The process according to at least one of the preceding claims, wherein contaminated propanediol obtained by hydrogenating a glycerol-containing stream in the presence of a catalyst is purified.

## Revendications

1. Procédé pour la purification de 1,2-propanediol d'impuretés par distillation, **caractérisé en ce que** le propanediol contaminé
1. est d'abord passé au travers d'une colonne A à substances de bas point d'ébullition, qui comprend une partie d'enrichissement et une partie d'épuisement, la colonne A à substances de bas point d'ébullition présentant, dans la partie d'enrichissement, moins de 20 étages théoriques de séparation et, dans la partie d'épuisement, moins de 40 étages théoriques de séparation, le rapport de reflux étant inférieur à 5 et la température du fond étant inférieure à 200°C
1.1 les substances de bas point d'ébullition, en particulier le méthanol, le 1-propanol, le 2-propanol, l'eau ainsi que les substances odoriférantes de bas point d'ébullition, étant séparées via la tête de la colonne et
1.2 un flux de 1,2-propanediol, qui est constitué par au moins 97% en poids de 1,2-propanediol, contenant encore des impuretés, étant soutiré du fond de la colonne, qui
2. est introduit dans une colonne B pour la distillation pure
2.1 du 1,2-propanediol purifié, peu odorant, quittant la colonne comme soutirage latéral liquide
2.2 un flux de préférence faible de 1,2-propanediol encore contaminé, contenant des substances odoriférantes étant soutiré via la tête de la colonne B et
2.3 des substances de point d'ébullition élevé, tels que l'éthylèneglycol et le glycérol, étant séparées via le fond,
le 1,2-propanediol à purifier provenant du produit d'hydrogénation évacué, qui est formé lors de l'hydrogénation de glycérol en 1,2-propanediol.

2. Procédé selon au moins l'une quelconque des revendications précédentes, **caractérisé en ce qu'**il existe, dans la colonne de distillation pure B, dans la partie d'enrichissement au-dessus du soutirage latéral liquide, moins de 60 étages théoriques de séparation, entre le site d'alimentation et le soutirage latéral liquide, moins de 120 étages théoriques de séparation et, dans la partie d'épuisement, moins de 60 étages théoriques de séparation et **en ce que** le rapport de reflux, par rapport à l'alimentation de la colonne de distillation pure, en tête de la colonne, est inférieur à 10 et la température du fond est inférieure à 200°C.

3. Procédé selon au moins l'une quelconque des revendications précédentes, **caractérisé en ce que** le propanediol contaminé, introduit dans la colonne A est contaminé par des thiols, des acides gras, des esters, des aldéhydes et/ou des cétones.

4. Procédé selon au moins l'une quelconque des revendications précédentes, **caractérisé en ce que** le propanediol purifié, après soutirage de la colonne de distillation pure B, est constitué, à raison d'au moins 99% en poids, de 1,2-propanediol pur et, à raison d'au maximum 0,01% en poids, de thiols, d'acides gras, d'esters, d'aldéhydes et/ou de cétones et, à raison d'au maximum 0,1% en poids, d'éthylèneglycol.

5. Procédé selon au moins l'une quelconque des revendications précédentes, **caractérisé en ce qu'**on purifie un propanediol contaminé qui a été obtenu par hydrogénation d'un flux contenant du glycérol en présence d'un catalyseur.
